(19) Europäisches Patentamt
European Patent Office
Office européen des brevets

(11) **EP 1 740 974 B1**

(12) **FASCICULE DE BREVET EUROPEEN**

(45) Date de publication et mention
de la délivrance du brevet:
**01.09.2010 Bulletin 2010/35**

(51) Int Cl.:
***G01S 15/89*** *(2006.01)*   ***G02B 21/00*** *(2006.01)*
***G01F 1/00*** *(2006.01)*

(21) Numéro de dépôt: **05744612.2**

(22) Date de dépôt: **25.03.2005**

(86) Numéro de dépôt international:
**PCT/FR2005/000718**

(87) Numéro de publication internationale:
**WO 2005/098474 (20.10.2005 Gazette 2005/42)**

(54) **PROCEDE ET SYSTEME DE MESURE DE VITESSE DU FLUX SANGUIN**

VERFAHREN UND SYSTEM ZUR MESSUNG DER GESCHWINDIGKEIT EINER BLUTSTRÖMUNG

METHOD AND SYSTEM FOR MEASURING THE SPEED OF BLOOD FLOW

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **02.04.2004 FR 0403519**

(43) Date de publication de la demande:
**10.01.2007 Bulletin 2007/02**

(73) Titulaire: **Mauna Kea Technologies
75010 Paris (FR)**

(72) Inventeurs:
• **LACOMBE, François**
**F-92370 Chaville (FR)**
• **LE GOUALHER, Georges**
**F-35520 La Mézière (FR)**
• **PERCHANT, Aymeric**
**F-94120 Fontenay sous Bois (FR)**
• **AYACHE, Nicholas**
**F-06000 Nice (FR)**

(74) Mandataire: **Pontet, Bernard et al
Pontet Allano & Associés s.e.l.a.r.l.
25, Rue Jean Rostand
Parc Club Orsay Université
91893 Orsay Cedex (FR)**

(56) Documents cités:
**WO-A-2004/051310     US-A- 6 061 176
US-B1- 6 473 698**

**Description**

La présente invention se rapporte à un procédé pour mesurer la vitesse d'un objet microscopique en mouvement au sein d'un écoulement, tel qu'un flux sanguin, à partir d'un microscope à balayage lumineux.

Elle s'applique notamment mais non exclusivement à l'étude de la microcirculation dans lequel la problématique est de détecter des particules telles que des globules rouges ou des leucocytes en déplacement, d'estimer le sens de déplacement de ces particules ainsi que leurs vitesses.

La présente invention peut toutefois s'appliquer à d'autres domaines tels que par exemple la microfluidique.

L'estimation de la vitesse de déplacement d'un objet à partir d'un système d'imagerie se fait classiquement à partir d'une séquence d'images représentant cet objet en mouvement. L'hypothèse de base étant que la cadence d'acquisition du système d'imagerie est telle que l'objet effectue de petits déplacements d'une image à l'autre. La présence de l'objet, à différentes positions, sur la série temporelle d'images permet alors de remonter, moyennant un étalonnage du système d'acquisition, à la vitesse de ce dernier.

On connaît le document "Erythrocyte velocity measurement in microvessels by a two-slit photometric method" de H. Wayland et RC. Johnson, publié dans J. Appl. Physiol. 22(2) :333-337, 1967, et décrivant une méthode photométrique à deux fentes. La mesure par méthode photométrique à deux fentes est probablement la technique la plus ancienne de mesure automatisée de vitesse du flux sanguin. Cette méthode mesure la vitesse des globules rouges et s'applique de préférence aux capillaires et aux petites veinules dans lesquelles les globules circulent en file, isolés ou par petits agrégats. La mesure se fait sur une séquence vidéo, à l'aide de deux fentes placées sur l'écran diffusant la séquence. Les deux fentes sont parallèles et orientées perpendiculairement au vaisseau sur lequel est réalisée la mesure. Une photodiode est présente en face de chacune des deux fentes. L'appareil utilisé propose deux modes de mesures. Dans un premier mode, les deux fentes sont espacées (en distance équivalente sur le tissu) de 45 $\mu$m à 70 $\mu$m. La mesure de vitesse est réalisée par un calcul de l'inter-corrélation des deux signaux issus des photodiodes. Dans le second mode de mesure, les deux fentes sont très rapprochées 7.4 $\mu$m en distance équivalente tissu, soit une distance légèrement inférieure au diamètre moyen d'un globule rouge. En conséquence, deux signaux consécutifs générés respectivement par la diode amont et la diode aval sont causés par un même globule, et il est donc possible de calculer la vitesse de ce globule. Cependant, les limitations de la méthode sont la nécessité de faire la mesure sur des vaisseaux très fins afin de limiter l'observation à un globule rouge, et les vitesses mesurées, qui en raison de la fréquence image de 30 images par seconde, ne peuvent dépasser 2 mm/s.

On connaît également le méthode de la projection Spatio-Temporelle ("Line shift Diagram"). Cette méthode est une extension de la précédente. Au lieu d'échantillonner le signal en deux points, l'utilisateur sélectionne une zone d'intérêt, c'est à dire un rectangle inscrit dans un vaisseau. A chaque image, une moyenne des niveaux de gris est calculée sur la largeur du vaisseau en chaque point de l'axe du vaisseau, le signal de la zone d'intérêt est projeté en une dimension sur l'axe du vaisseau. Ensuite les signaux unidimensionnels obtenus pour chaque image sont alignés verticalement pour donner une image spatiotemporelle qui laisse apparaître les traces des globules. La vitesse est évaluée en corrélant les signaux adjacents. Cette méthode est utilisée par les logiciels CapImage® et Capiscope®, de pair avec l'appareil d'acquisition Cytoscan®. Une telle méthode est notamment décrite dans le document "Orthogonal Polarisation Spectral Imagina : A new method for study of microcirculation." de W. Groner, I.W. Winkelman, A.G. Harris, G. Inde, G.I. Bouma, K. Messmer, et R.G. Nadeau, publié dans Nature Medecine, 5 :1209-1213, 1999. La principale limitation est la gamme de vitesse mesurée, qui ne peut dépasser 2 mm/s en raison de la fréquence des images (25 images/secondes ou 50 images/secondes si les mesures sont réalisées alternativement sur les deux champs entrelacés du flux vidéo).

Le SLO pour "Scanning Laser Ophtalmoscope" en langue anglaise, est un appareil dont le principe repose sur la microscopie confocale non fibrée. Les images sont capturées à une cadence de 50 images entrelacées par seconde. La méthode de mesure de vitesse utilisée par cet appareil repose sur le suivi de cellule. Une même image est constituée de champs entrelacés correspondants à deux instants espacés de 20 ms, un globule en mouvement apparaît donc à deux endroits différents sur l'image, une fois sur les lignes paires et une autre sur les lignes impaires. Une fois les deux images du globule localisées, la mesure de la vitesse est immédiate. Le grand champ de l'appareil (jusqu'à 1200 $\mu$m permet de mesurer des vitesses de plusieurs cm/s). Les limitations de cet appareil sont principalement dues au suivi des globules : il faut des globules marqués de grosse taille (12 $\mu$m pour les leucocytes) et en faible nombre. Une telle méthode semble inapplicable à la mesure de vitesse de globules rouges, dont les dimensions sont plus petites (7 - 8 $\mu$m), de concentration beaucoup plus forte (1 000 fois plus importante que celle des globules blancs) et plus difficiles à marquer.

Une autre méthode de mesure qui est couplée couramment avec le Cytoscan® ou le SLO est la mesure de vitesse de globules rouges par effet Doppler. L'effet Doppler décrit le décalage fréquentiel que subit une onde réfléchie par un objet en mouvement par rapport à l'observateur. Dans le cas du flux sanguin, les objets en mouvements sont les globules rouges. Une onde monofréquentielle (par exemple un laser) de longueur d'onde donnée est envoyée sur un vaisseau sanguin. La mesure de la vitesse par effet Doppler a l'avantage d'être très rapide et précise. Suivant le matériel et les logiciels d'analyse utilisés les vitesse maximales mesurables varient de 1 mm/s à plusieurs cm/s. Un inconvénient du Doppler vient de la difficulté à identifier précisément la zone sur laquelle la vitesse est mesurée, surtout en profondeur. La lumière réfléchie peut provenir de différents vaisseaux où le sang circule à des vitesses différentes.

On connaît encore la méthode par Analyse Spatio-Temporelle. Une telle méthode pour la mesure de la vitesse de leucocytes est décrite dans le document "Measuring microcirculation using spatiotemporal image analysis." par Yoshinobu Sato et al., CVRMed, pages 302-308, 1995. Cette analyse comporte trois étapes principales : i) Dans un premier temps, le vaisseau est extrait de l'image par une segmentation faite sur l'histogramme des variances temporelles des pixels des trames de la séquence. ii) Une image spatiotemporelle est alors construite à partir des images successives du vaisseau. Cette image peut être tridimensionnelle, ou bidimensionnelle si chaque image de la séquence est projetée sur l'axe ou les contours du vaisseau. iii) Les traces laissées dans l'image spatiotemporelle par le mouvement des leucocytes sont ensuite renforcées par application d'un banc de filtres à orientation sélective (par exemple un banc de filtres de Gabor). Les traces sont extraites par seuillage des meilleures réponses. La dernière étape consiste à connecter entre elles les traces pour reconstituer les trajectoires entières des leucocytes. L'obtention de ces traces permet ensuite par un calcul de leurs tangentes d'avoir une estimation de la vitesse des leucocytes sur leurs trajectoires.

Le document US 6 473 698 décrit une méthode selon le préambule de la revendication 1.

Egalement dans le domaine de l'analyse spatio-temporelle, on connaît les documents :

- "Two-photon imaging of neocortical microcirculation." D. Kleinfeld and W. Denk; In Imaging Neurons: A Laboratory Manual (R. Yuste, F. Lanni, and A. Konnerth, editors), 1999, Cold Spring Harbor Laboratory Press, NY, pp. 23.1-23.15; accessible sur Internet à l'adresse suivante : http://physics.ucsd.edu/neurophysics/publications/kleinfeld_denk_c shl 2003.pdf; et
- "Two-photon imaging of capillary blood flow in olfactory bulb glomeruli" E. Chaigneau et al., PNAS, 28 Octobre, 2003; 100(22): 13081 - 13086; accessible sur Internet à l'adresse suivante : http://www.pnas.org/cgi/content/full/100/22/13081.

Ces documents décrivent une méthode de mesure de vitesse en réalisant, à l'aide d'un microscope non fibré, plusieurs balayages successifs sur l'axe du vaisseau sanguin. On obtient donc plusieurs images "unidimensionnelles" d'un même segment à des instants différents successifs. Ces images sont mises bout à bout de façon à former une visualisation globale dont l'ordonnée est un repère temporel. Le mouvement des particules dans le vaisseau sanguin se traduit sous forme de bandes obliques. On détermine la vitesse instantanée en calculant la pente de chaque bande sur la visualisation globale. Toutefois, l'inconvénient de cette méthode réside sur le fait qu'il est indispensable de positionner le dispositif d'acquisition parallèlement à l'axe du vaisseau sanguin. Par ailleurs, la constitution de la visualisation globale nécessite l'acquisition de plusieurs images d'un même segment.

Dans la plupart des techniques qui viennent d'être présentées, la mesure de la vitesse repose sur une analyse entre au moins deux images successives d'une acquisition; ce qui par ailleurs soulève des problèmes de bougé et de mise en correspondance entre images. Dès lors, la gamme de vitesse mesurable par ces techniques dépend du champ de vue ainsi que de la fréquence image. Or, pour des systèmes d'imagerie à balayage présentant un petit champ de vue, par exemple de l'ordre de 166 $\mu$m x 118 $\mu$m, et des cadences d'acquisition, par exemple de l'ordre de 12 images par seconde, une particule animée d'une vitesse supérieure à 1.8 mm/s traversera le champ d'observation entre deux images successives, ce qui rend impossible l'utilisation de la plupart des techniques citées précédemment.

La présente invention vise à remédier aux inconvénients précités en proposant un procédé apte à mesurer la vitesse des globules rouges notamment.

La présente invention a pour but la mesure de vitesse de particules en mouvement rapide au moyen d'un système d'imagerie à balayage. Par mouvement rapide, on entend une vitesse au delà d'environ 2mm/s.

On atteint le but recherché avec un procédé pour mesurer la vitesse d'un objet microscopique, tel que par exemple des globules rouges et des leucocytes en mouvement au sein d'un écoulement, tel qu'un flux sanguin, à partir d'un microscope à balayage lumineux. Selon l'invention, ce procédé comprend les étapes suivantes :

- acquisition d'une image par balayage lumineux en x et y d'un plan contenant ledit objet, ce plan est aussi appelé champ imagé subsurfacique situé à quelques $\mu$m dans un échantillon ou un tissu biologique;
- détection dans le plan (x, y) d'une strie engendrée par le déplacement dudit objet lors de l'acquisition de ladite image;
- détermination de la pente de ladite strie dans le plan (x, y) ;
- estimation de la vitesse $V_g$ dudit objet à partir de la pente ainsi déterminée.

On peut utiliser un microscope, confocal ou non, à balayage lumineux, notamment laser, en mode fibré ou non fibré. Pour mettre en oeuvre le mode de réalisation non fibré, on peut utiliser, en adaptant l'électronique de traitement, un appareil de type SLO ou tout autre appareil d'acquisition d'image à balayage (x, y) pour lequel les vitesses de balayage sont adaptées à la mise en oeuvre du procédé selon l'invention.

En complément de ce qui précède, on peut utiliser de façon non limitative un microscope, confocal ou non, laser fibré, notamment mono-fibre, à balayage distal. Ce balayage distal peut être réalisé par un micromiroir, par déplacement de lentilles ou d'optiques, par balayage spectral... On peut aussi utiliser un système mono-fibré à balayage proximal où le balayage lumineux est obtenu par déviation d'une extrémité de la fibre optique au sein d'une tête optique proche de l'objet observé.

Contrairement aux méthodes d'analyse spatiotemporelle de Kleinfeld et de Chaigneau où l'on acquiert une succession d'images "unidimensionnelles" d'un même segment dans l'axe du vaisseau sanguin, dans la présente invention, on peut utiliser une seule image acquise par un balayage plan bidimensionnel.

Contrairement à l'art antérieur où l'on mesure des stries sur une image spatio-temporelle (t=f(x)), ici les stries sont complètement différentes puisqu'elles sont issues d'une image plan (y=f(x)), la notion temporelle étant induite dans le système de balayage point à point en "Z". On se sert avantageusement d'une image morphologique. Par exemple, dans le document de Yoshinobu Sato selon l'art antérieur, pour obtenir des stries, on réalise plusieurs images dans lesquelles on extrait une seule ligne dans chaque image, et on élabore ainsi l'image finale t=f(x).

L'utilisation d'un système d'imagerie à balayage, système dans lequel l'objet en déplacement va être observé pendant le temps de génération d'une image (temps de parcours trame), conduit à l'apparition de stries permettant de remonter à une estimation de la vitesse d'objets en déplacement à partir d'une seule image. Ces stries proviennent donc de l'interaction entre l'objet en mouvement et le système de balayage. L'invention est notamment remarquable par le fait que ces stries sont généralement considérées par l'homme du métier comme des parasites à éliminer dans un système d'imagerie à balayage. On tire donc profit des éléments considérés comme des aberrations dans les microscopes à balayage laser, notamment en mode fibré. Pour ce faire, l'invention comporte une étape de détection de stries dans laquelle on réalise les étapes suivantes :

- accentuation d'un ensemble de stries de l'image par application d'un filtre;
- application d'un seuil de façon à conserver les stries les plus importantes;
- ajustement d'une droite ou d'une ellipse sur chacune de ces stries; et
- identification de ladite strie.

Selon l'invention, compte tenu des ordres de grandeurs des vitesses de balayage et de celles des objets observés, il est possible de simplifier le modèle de la trajectoire du spot lumineux. Une première simplification consiste à considérer la trajectoire du spot comme horizontale sur la fenêtre d'acquisition. Cette simplification est justifiée par le rapport mille existant entre la vitesse horizontale et la vitesse verticale du spot, le calcul confirme que la position verticale du spot varie de moins de 0.1 $\mu$m sur la fenêtre d'acquisition.

Une seconde approximation est de considérer le temps nécessaire au spot pour couvrir horizontalement la fenêtre d'acquisition comme négligeable, c'est à dire de considérer les objets comme immobiles pendant le parcours d'une ligne de balayage. La vitesse des objets observés (dans les vaisseaux considérés, les globules rouges ont une vitesse inférieure à 20 mm/sec) par rapport à la vitesse horizontale du spot (> 1 m/s) justifie cette approximation.

Au final le balayage est modélisé par des lignes de balayage horizontal instantané espacées spatialement d'une distance $V_y/f_x$, avec $f_x$ fréquence du balayage "x".

Une fois le balayage modélisé, il reste à modéliser les objets en mouvement, par exemple dans la suite les globules rouges. Plusieurs modèles ont été envisagés pour décrire les globules rouges : du simple bâtonnet à un modèle tridimensionnel réaliste.

Le modèle le plus simple pouvant rendre compte des déformations observées consiste à représenter un globule rouge par un bâtonnet vertical. Dans le cas de l'utilisation de ce modèle, on obtient une relation simple liant l'angle $\alpha$ des stries observées (par rapport à l'axe "x") à la vitesse verticale du balayage $V_y$ et à la vitesse de déplacement horizontal $V_g\cos(\theta)$ du globule :

$$\tan\alpha = \frac{V_y}{V_g \cos\theta} \qquad\qquad (1)$$

avec $V_y$ la vitesse verticale du spot lumineux utilisé pour le balayage, $V_g$ la vitesse du globule recherché, $\theta$ l'angle entre le vecteur $V_g$ et l'axe x de balayage rapide. Tan $(\alpha)$ est la pente des stries.

Si la trajectoire des globules est supposée colinéaire au bords du vaisseau véhiculant l'objet alors il est possible de connaître $\theta$ en détectant les bords du vaisseau et donc d'accéder à la vitesse $V_g$ du globule.

Dans le cas général où 9 est inconnu mais où la longueur L de la strie est connue, deux possibilités sont envisageables :

1) La taille D du globule est connue.

Dans ce cas, on considère $t_v$ le temps de visibilité du globule, défini comme le temps écoulé entre la première intersection et la dernière intersection des trajectoires. Le globule a une vitesse verticale $V_g \sin(\theta)$ et une étendue verticale D. On assimile donc l'objet à une barre perpendiculaire à la direction d'écoulement, de longueur finie suffisamment grande pour s'étaler sur plusieurs lignes de balayage horizontal. Le spot est ponctuel et a une vitesse verticale $V_y$, $t_v$ dépend donc uniquement de la différence de vitesse verticale du spot et du globule, et de D selon la relation :

$$t_v = \frac{D}{\left|V_y - V_g \sin\theta\right|} \qquad (2)$$

Les étendues verticale $L|\sin(\alpha)|$ et horizontale $L|\cos(\alpha)|$ de la strie observée sont directement reliées à $t_v$ et aux vitesses verticale du spot et horizontale du globule par :

$$L\left|\sin\alpha\right| = t_v\left|V_y\right| \qquad (3)$$

$$L\left|\cos\alpha\right| = t_v V_g\left|\cos\theta\right|$$

Les relations 2 et 3 permettent d'écrire :

$$L = \frac{D}{\left|V_y - V_g \sin\theta\right|}\sqrt{V_y^2 + V_g^2 \cos^2\theta} \qquad (4)$$

Ce qui, combiné à la relation (1), donne un système de deux équations à deux inconnues $\theta$ et $V_y$. La résolution doit se faire selon le signe de $V_y - V_g\sin(\theta)$, c'est à dire selon que le spot se déplace plus ou moins vite verticalement que le globule.

Dans le cas où les deux vitesses sont égales, le temps de visibilité devient infini et la strie devient infiniment longue. On a alors $V_g\sin(\theta) = V_y$, en remplaçant dans la relation (1), il vient $\tan(\alpha) = \tan(\theta)$. Par conséquent, la strie est exactement la trajectoire du globule et la vitesse $V_g$ est donnée par :

$$V_g = \left|\frac{V_y}{\sin\alpha}\right| \qquad (5)$$

Si le spot lumineux se déplace verticalement plus vite que le globule ($V_y > V_g\sin(\theta)$), alors la résolution du système (3) donne :

$$\tan\theta = \tan\alpha - \frac{D}{L\cos\alpha} \qquad (6)$$

$$V_g = \left|V_y\right| \sqrt{\left(1 - \frac{D}{L\sin\alpha}\right)^2 + \frac{1}{\tan^2\alpha}}$$

Si le spot lumineux se déplace verticalement moins vite que le globule ($V_y < V_g \sin(\theta)$), alors la résolution du système (3) donne

$$\tan\theta = \tan\alpha + \frac{D}{L\cos\alpha} \qquad (7)$$

$$V_g = \left|V_y\right| \sqrt{\left(1 + \frac{D}{L\sin\alpha}\right)^2 + \frac{1}{\tan^2\alpha}}$$

2) Lorsqu'un balayage inversé est disponible :

Si l'on dispose d'un autre balayage, inversé par rapport au précédent, c'est à dire de vitesse verticale $-V_y$, une strie d'angle $-\alpha$ et de longueur L' sera observée. L' est donné par :

$$L' = \frac{D}{\left|-V_y - V_g\sin\theta\right|} \sqrt{V_y^2 + V_g^2\cos^2\theta} \qquad (8)$$

Le rapport des relations (4) et (8) permet d'écrire :

$$\frac{L}{L'} = \frac{\left|V_y + V_g\sin\theta\right|}{\left|V_y - V_g\sin\theta\right|} \qquad (9)$$

deux cas sont alors à distinguer : $|V_y| > |V_g\sin(\theta)|$ et $|V_y| < |V_g\sin(\theta)|$ (si $|V_y| = |V_g\sin(\theta)|$, alors la relation (5) s'applique).
Si $|V_y| > |V_g\sin(\theta)|$ alors la résolution donne :

$$\tan\theta = \tan\alpha \frac{L - L'}{L + L'}$$

$$(10)$$

$$V_g = \left|V_y\right| \sqrt{\left(\frac{L - L'}{L + L'}\right)^2 + \frac{1}{\tan^2\alpha}}$$

Si $|V_y| < |V_g\sin(\theta)|$ alors la résolution donne :

$$\tan \theta = \tan \alpha \frac{L + L'}{L - L'}$$

$$(11)$$

$$V_g = \left| V_y \right| \sqrt{\left(\frac{L+L'}{L-L'}\right)^2 + \frac{1}{\tan^2 \alpha}}$$

Dans tout ce qui précède, l'objet, tel qu'un globule rouge, est assimilé à un bâtonnet vertical de longueur D. L'homme du métier comprendra aisément qu'on peut remplacer cette hypothèse par celle, plus réaliste, d'une sphère (ou disque), voire d'une ellipse ou d'une structure plus compliquée encore et évaluer l'écart entre l'état observé dans ce cas et celui observé dans le cas simple. Selon la précision recherchée, il conviendra de prendre ou non en compte cet écart.

A titre d'exemple, le globule rouge peut être représenté sous la forme d'une sphère solide ou d'une forme biconcave possédant une symétrie de révolution. Dans les deux cas, on assimile la projection orthogonale dans le plan d'observation à un disque de rayon R. Sur l'image acquise, ce disque en mouvement apparaît comme une ellipse dont l'angle $\alpha$ entre la strie et l'axe "x" est donné par :

$$\tan(2\alpha) = \frac{2\cos(\theta)}{\dfrac{V_g}{V_y} - 2\sin(\theta)}$$

$$(12)$$

La longueur de l'axe principal est donnée par :

$$L = \frac{2R\sqrt{2}}{\sqrt{V_r{}^2 - 2V_r \sin(\theta) + 2 - |V_r|\sqrt{V_r{}^2 - 4V_r \sin(\theta) + 4}}}$$

$$(13)$$

avec $V_r = \dfrac{V_g}{V_y}$ et R le rayon du disque considéré.

Des simulations numériques effectuées avec des modèles plus complexes de représentation des globules rouges ( surfaces toroïdales) montrent que le modèle du disque est suffisant pour représenter les globules rouges.

Si l'orientation $\theta$ de la trajectoire du globule est inconnue, mais la longueur L des stries est disponible, il est possible de remonter partiellement à une information de vitesse. Les relations (12) et (13) forment un système liant le couple ($\alpha$, L) qui sont les paramètres observables lors des acquisitions au couple ($\theta$, $V_g$) qui sont les paramètres recherchés.

Selon un autre aspect de l'invention, il est proposé un système de microscopie confocale à balayage lumineux en mode fibré, utilisé pour mesurer la vitesse de l'objet microscopique en mouvement au sein d'un écoulement, tel qu'un flux sanguin, ce système comprenant :

- des moyens pour acquérir une image par balayage lumineux en x et y d'un plan contenant ledit objet;
- des moyens pour détecter une strie engendrée par le déplacement dudit objet lors de l'acquisition de ladite image;
- des moyens pour déterminer la pente de ladite strie; et
- des moyens pour estimer la vitesse $V_g$ dudit objet à partir de la pente ainsi déterminée.

D'autres avantages et caractéristiques de l'invention apparaîtront à l'examen de la description détaillée d'un mode de mise en oeuvre nullement limitatif, et des dessins annexés, sur lesquels :

- la figure 1 est un schéma général d'un exemple de système d'imagerie confocale fibrée mettant en oeuvre le procédé selon l'invention;
- la figure 2 est un schéma très simplifié illustrant le mode de balayage du système d'imagerie de la figure 1;
- la figure 3 est une image schématique représentant des stries, cette image étant issue d'une acquisition simulée; et
- la figure 4 est une vue schématique d'une étape de sélection de stries.

Bien que l'invention n'y soit pas limitée, on va maintenant décrire le procédé selon l'invention mis en oeuvre dans un microscope confocal à balayage laser en mode fibré, ce procédé s'appliquant au domaine de la microcirculation, dont les ordres de grandeurs sont les suivantes :

- les artérioles ont un diamètre qui varie entre 50 $\mu$m et 100 $\mu$m; les capillaires sont beaucoup plus fins avec un diamètre de 3 $\mu$m à 8 $\mu$m; enfin les veinules ont un diamètre de 30 $\mu$m à 50 $\mu$m;
- la vitesse des globules rouges dans ces vaisseaux est comprise dans une gamme allant de moins de 1 mm/s pour les vaisseaux les plus petits à quelques dizaines de mm/s pour les artérioles;
- les globules rouges sont des cellules dont le diamètre moyen est d'environ 7 $\mu$m, à comparer avec des diamètres de 10 $\mu$m à 15 $\mu$m pour les globules blancs.

D'une manière générale, pour mettre en oeuvre la présente invention, on peut se baser sur le système décrit dans le document WO 2004/008952A1, « Procédé et appareillage d'imagerie de fluorescence haute résolution par fibre optique et notamment d'imagerie confocale », Mauna Kea Technologies, dans lequel on utilise un guide d'image fait de plusieurs milliers de fibres optiques, un signal d'excitation étant émis par une source, dévié et injecté tour à tour dans l'une des fibres dudit guide, chaque point d'excitation du tissu en sortie de fibre émettant en retour un signal de fluorescence collecté par ladite fibre, puis détecté et numérisé pour former un élément d'image. Selon un premier aspect, le procédé décrit dans ce document WO2004/008952A1 prévoit la focalisation du faisceau en sortie de fibre pour exciter un plan subsurfacique et réaliser une image confocale. Selon un second aspect, le procédé prévoit de produire un faisceau divergent en sortie de fibre susceptible d'exciter un microvolume du tissu depuis la surface. Le signal d'excitation est dévié à une vitesse correspondant à l'acquisition d'un nombre d'images par seconde suffisant pour une utilisation en temps réel et l'on détecte le signal de fluorescence à une fréquence de détection correspondant à une fréquence minimale d'échantillonnage des fibres une à une.

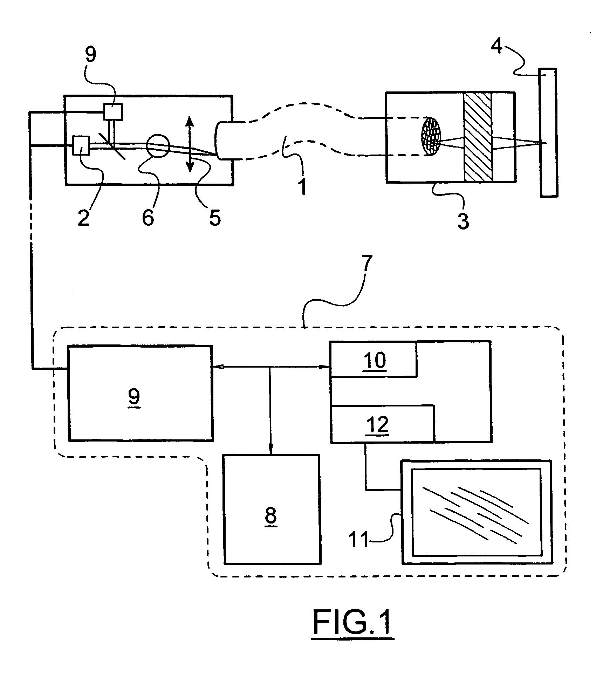

Sur la figure 1 on voit un faisceau ordonné de fibres optiques souples formant un guide d'image 1 avec, sur son extrémité proximale, une source lumineuse 2 et un système d'injection de fibres permettant d'illuminer les fibres une à une et, sur son extrémité distale, une tête optique 3 permettant de focaliser le faisceau sortant de la fibre illuminée en un point situé à une profondeur donnée de l'objet observé 4. Le système d'injection comprend plusieurs éléments optiques 5 précédés d'un système de balayage de fibres 6, tel qu'un déviateur, permettant de balayer les fibres une à une à très grande vitesse. Chaque fibre est utilisée tour à tour pour véhiculer le faisceau d'illumination et également le faisceau de retour correspondant provenant de l'objet observé. La résolution spatiale est obtenue par focalisation du faisceau laser en un point et par le caractère confocal résidant dans le filtrage spatial de l'objet observé par les mêmes fibres que celles ayant servi à l'illumination. Cela permet de réceptionner, au moyen d'un photodétecteur 9, exclusivement le signal provenant de l'objet observé et de réaliser une image point par point.

Le guide d'image 1 est constitué d'un très grand nombre de fibres optiques souples, par exemple 30 000 fibres de 2 $\mu$m de diamètre et espacées de 3,3 $\mu$m. En pratique, on peut utiliser soit l'ensemble des fibres du guide d'image, soit un sous-ensemble choisi de ces fibres, par exemple centré.

Les moyens électroniques et informatiques 7 de commande, d'analyse et de traitement numérique du signal détecté et de visualisation comprennent notamment les cartes suivantes:

- une carte de synchronisation 8 qui a pour fonctions :
- de commander de manière synchronisée le balayage;
- de connaître à tout instant la position du spot laser ainsi balayé ; et
- de gérer toutes les autres cartes par l'intermédiaire d'un microcontrôleur lui-même pouvant être piloté ;
- une carte détecteur 9 qui comprend un circuit analogique qui réalise notamment une adaptation d'impédance, un convertisseur analogique numérique puis un composant logique programmable (par exemple un circuit FPGA) qui met en forme le signal ;
- une carte d'acquisition numérique 10 qui permet de traiter un flot de données numériques à fréquence variable et de l'afficher sur un écran 11;
- une carte graphique 12.

En variante, on peut utiliser une seule carte regroupant les fonctionnalités de ces différentes cartes.

Ces moyens électroniques et informatiques 7, aptes à réaliser les étapes du procédé selon l'invention, peuvent se présenter sous forme d'un micro ordinateur doté de moyen de traitement nécessaire pour calculer la vitesse des globules rouges.

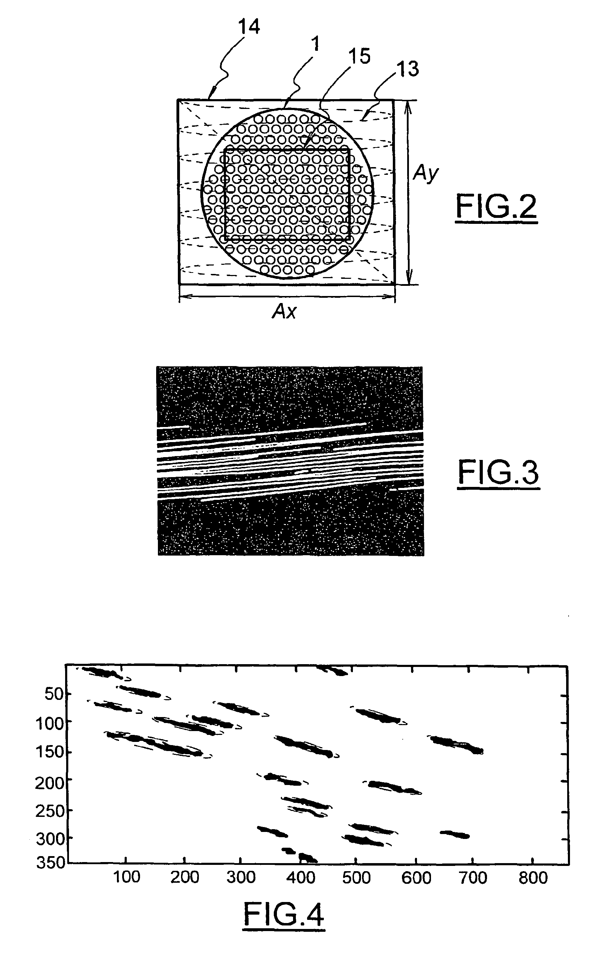

La figure 2 est un schéma très simplifié illustrant le mode de balayage du système d'imagerie de la figure 1. Le spot laser de balayage est symbolisé par les pointillés 13 qui décrivent un trajectoire de balayage classique dans une fenêtre carrée de balayage 14. La trajectoire du spot laser 13 est un "Z" de haut en bas. La vitesse horizontale $V_x$ selon l'axe horizontale $A_x$ est supposée très supérieure devant la vitesse $V_y$ selon l'axe $A_y$. Cette hypothèse revient à négliger le temps de rattrapage du globule par le spot entre deux balayage horizontaux. C'est aussi confondre l'effet observé sur les lignes paires avec celui observé sur les lignes impaires. A titre d'exemple, la vitesse $V_y$ peut être de 3 mm/s, alors que celle de $V_x$ peut être de 5 m/s.

Sur la figure 2, à l'intérieur de la fenêtre de balayage 14, est également représenté le guide d'image 1 selon une vue en coupe transversale. Les fibres optiques sont représentées sous forme de cercles ordonnés. La zone imagée 15 ne correspond qu'à un nombre limité de fibres optiques situées à l'intérieur d'un rectangle. Le faisceau laser est successivement injecté dans chacune des fibres optiques. L'image représentée sur la figure 3, simule une acquisition, c'est à dire que chaque fibre n'a été injectée qu'une seule fois. Cette image fait apparaître des stries obliques correspondant à l'interaction entre le système de balayage et les particules en mouvement.

L'apparition de ces stries s'explique par l'interaction entre l'image des globules rouges et le mécanisme de formation de l'image. Le spot laser effectue le balayage selon une trajectoire en forme de Z, une mesure est effectuée en un ensemble de positions du laser, par exemple 896 mesures par lignes sur 640 lignes. Un globule rouge en mouvement va être intersecté à une position donnée sur une ligne du balayage. A la ligne suivante, ce globule est encore intersecté, il s'est cependant déplacé par rapport à la ligne précédente. Ce phénomène continue tant que l'intersection entre la ligne de balayage et le globule existe. Ce phénomène créé alors une strie dont l'inclinaison est fonction de la vitesse du globule rouge.

Sur la figure 4, après acquisition d'une image, les stries sont mises en évidence. Un seuillage permet de ne conserver que les stries les plus importantes. Chaque strie est ensuite encadrée par une ellipse permettant de définir une pente. Puis, les moyens électroniques et informatiques 7 déterminent la pente de chaque strie de façon à calculer la vitesse de chaque globule rouge.

La présente invention permet donc de déterminer la vitesse à partir d'une seule image. Ceci permet d'éviter notamment des problèmes de bougés de mise en correspondance d'images. Elle permet avantageusement d'appréhender des vitesses importantes en regard du champ de vue et de la fréquence d'acquisition. A titre d'exemple, un système d'acquisition d'image à 11 Hz permet d'appréhender des vitesses de l'ordre de 5 à 25 mm/sec, soit des vitesses impossibles à estimer avec la plupart des techniques de l'art antérieur.

De préférence, on mesure la vitesse de globule dont le mouvement vertical ne va pas à l'encontre du spot. De manière général l'angle de la trajectoire doit se situer entre l'horizontale et l'angle critique pour lequel la vitesse verticale du globule devient égale à celle du spot.

Bien sûr, l'invention n'est pas limitée aux exemples qui viennent d'être décrits et de nombreux aménagements peuvent être apportés à ces exemples sans sortir du cadre de l'invention. On peut notamment envisager des mesures faites sur plusieurs stries, voire sur plusieurs images afin d'améliorer la précision.

## Revendications

1. Procédé pour mesurer la vitesse d'un objet microscopique en mouvement au sein d'un écoulement, tel qu'un flux sanguin, à partir d'un microscope à balayage lumineux, ce procédé comprenant les étapes suivantes :

    - acquisition d'une image par balayage lumineux en x et y d'un plan contenant ledit objet;
    - détection dans le plan (x, y) d'une strie engendrée par le déplacement dudit objet lors de l'acquisition de ladite image; le procédé étant **caractérisé en ce qu'**il comprend les étapes consistant en
    - la détermination de la pente de ladite strie dans le plan (x, y); et
    - l'estimation de la vitesse $V_g$ dudit objet à partir de la pente ainsi déterminée.

2. Procédé selon la revendication 1, **caractérisé en ce que** l'étape de la détection de la strie comprend les étapes suivantes

    - accentuation d'un ensemble de stries de l'image par application d'un filtre;
    - application d'un seuil de façon à conserver les stries les plus importantes;
    - ajustement d'une droite ou d'une ellipse sur chacune de ces stries; et
    - identification de ladite strie.

3. Procédé selon la revendication 1 ou 2, **caractérisé en ce que** la vitesse $V_g$ dudit objet est donnée par la relation suivante
$V_g$*cos(θ) = $V_y$/tan(α) avec $V_y$ la vitesse verticale du spot lumineux utilisé pour le balayage, et "α" l'angle entre l'axe horizontal "x" et la strie.

4. Procédé selon la revendication 3, **caractérisé en ce que** l'angle θ est obtenu par détection des bords de vaisseaux sanguins véhiculant l'objet.

5. Procédé selon la revendication 3, **caractérisé en ce que** pour obtenir l'angle θ, on assimile ledit objet à un bâtonnet vertical de hauteur finie D et on calcule l'angle θ à partir de la relation suivante :

$$L = \frac{D}{\left|V_y - V_g \sin\theta\right|}\sqrt{V_y^2 + V_g^2 \cos^2\theta}$$

où L est la longueur de la strie.

6. Procédé selon la revendication 5, **caractérisé en ce que** dans le cas où $V_g$*sin(θ) < $V_y$ :

$$\tan\theta = \tan\alpha - \frac{D}{L\cos\alpha}$$

$$V_g = \left|V_y\right|\sqrt{\left(1 - \frac{D}{L\sin\alpha}\right)^2 + \frac{1}{\tan^2\alpha}}$$

7. Procédé selon la revendication 5, **caractérisé en ce que** dans le cas où $V_g$*sin(θ) > $V_y$ :

$$\tan\theta = \tan\alpha + \frac{D}{L\cos\alpha}$$

$$V_g = \left|V_y\right|\sqrt{\left(1 + \frac{D}{L\sin\alpha}\right)^2 + \frac{1}{\tan^2\alpha}}$$

8. Procédé selon la revendication 3, **caractérisé en ce qu'**on acquiert une seconde image du même plan mais dans un balayage inversé, et on utilise la relation suivante :

$$L' = \frac{D}{\left|-V_y - V_g \sin\theta\right|}\sqrt{V_y^2 + V_g^2 \cos^2\theta}$$

où L' est la longueur de la strie lors du balayage inversé.

9. Procédé selon la revendication 8 et 5, **caractérisé en ce que** lorsque $|V_g$* sin(θ)| < $|V_y|$ :

$$\tan\theta = \tan\alpha\,\frac{L-L'}{L+L'}$$

$$V_g = \left|V_y\right|\sqrt{\left(\frac{L-L'}{L+L'}\right)^2 + \frac{1}{\tan^2\alpha}}$$

où L est la longueur de la strie dans un premier sens de balayage, et L' la longueur de la strie lors du balayage inversé.

**10.** Procédé selon la revendication 5 et 8, **caractérisé en ce que** lorsque $|V_g{}^*\sin(\theta)\,| > |V_y|$:

$$\tan\theta = \tan\alpha\,\frac{L+L'}{L-L'}$$

$$V_g = \left|V_y\right|\sqrt{\left(\frac{L+L'}{L-L'}\right)^2 + \frac{1}{\tan^2\alpha}}$$

**11.** Procédé selon la revendication 3, **caractérisé en ce que** lorsque $V_g{}^*\sin(\theta) = V_y$, pour déterminer $V_g$ et $\theta$ on utilise en outre la relation suivante :

$$V_g = \left|\frac{V_y}{\sin\alpha}\right|$$

**12.** Procédé selon la revendication 3, **caractérisé en ce que** ledit objet étant un globule rouge, on assimile sa forme sur l'image acquise à une ellipse de rayon R dont l'angle $\alpha$ entre la strie et l'axe "x" est donné par :

$$\tan(2\alpha) = \frac{2\cos(\theta)}{\dfrac{V_g}{V_y} - 2\sin(\theta)}$$

et la longueur de l'axe principale est donnée par :

$$L = \frac{2R\sqrt{2}}{\sqrt{V_r^2 - 2V_r\sin(\theta) + 2} - |V_r|\sqrt{V_r^2 - 4V_r\sin(\theta) + 4}}$$

avec $V_r = \dfrac{V_g}{V_y}$

**13.** Procédé selon l'une quelconque des revendications précédentes, **caractérisé en ce qu'**on utilise un microscope confocal.

**14.** Procédé selon l'une quelconque des revendications précédentes, **caractérisé en ce qu'**on utilise un microscope à balayage lumineux en mode fibré.

**15.** Procédé selon l'une quelconque des revendications 1 à 13, **caractérisé en ce qu'**on utilise un microscope à balayage lumineux non fibré.

**16.** Système de microscopie à balayage lumineux, utilisé pour mesurer la vitesse d'un objet microscopique en mouvement au sein d'un écoulement, tel qu'un flux sanguin, ce système mettant en oeuvre un procédé selon l'une quelconque des revendications précédentes; ce système comprenant :

- des moyens pour acquérir une image par balayage lumineux en x et y d'un plan contenant ledit objet;
- des moyens pour détecter dans le plan (x, y) une strie engendrée par le déplacement dudit objet lors de l'acquisition de ladite image;
- des moyens pour déterminer dans le plan (x, y) la pente de ladite strie; et
- des moyens pour estimer la vitesse $V_g$ dudit objet à partir de la pente ainsi déterminée.

**17.** Système selon la revendication 16, **caractérisé en ce qu'**au cours de la détection de la strie, le système comprend :

- des moyens pour accentuer un ensemble de stries de l'image par application d'un filtre;
- des moyens pour appliquer un seuil de façon à conserver les stries les plus importantes;
- des moyens pour ajuster une ellipse sur chacune de ces stries; et
- des moyens pour identifier ladite strie.

**18.** Système selon la revendications 16 ou 17, **caractérisé en ce qu'**on utilise un microscope confocal.

**19.** Système selon l'une quelconque des revendications 16 à 18, **caractérisé en ce qu'**on utilise un microscope à balayage lumineux en mode fibré.

**20.** Système selon l'une quelconque des revendications 16 à 18, **caractérisé en ce qu'**on utilise un microscope à balayage lumineux non fibré.

**Claims**

**1.** Method for measuring the velocity of a microscopic object moving inside a flow, such as a blood flow, using a light-scanning microscope, this method comprising the following steps:

- acquisition of an image by x and y light scanning of a plane containing said object;
- detection in the plane (x, y) of a ridge produced by the movement of said object during the acquisition of said image; the method being **characterized by** the following steps:
- determination of the slope of said ridge in the plane (x, y); and
- estimation of the velocity $V_g$ of said object from the thus-determined slope.

**2.** Method according to claim 1, **characterized in that** the step of detection of the ridge comprises the following steps:

- enhancement of a group of ridges of the image by application of a filter;
- application of a threshold in order to retain the most significant ridges;
- fitting a straight line or an ellipse on each of these ridges; and
- identification of said ridge.

**3.** Method according to claim 1 or 2, **characterized in that** the velocity $V_g$ of said object is given by the following equation:

$V_g*\cos(\theta) = V_y/\tan(\alpha)$ with $V_y$ being the vertical velocity of the light spot used for the scanning, and "$\alpha$" the angle between the horizontal axis "x" and the ridge.

**4.** Method according to claim 3, **characterized in that** the angle $\theta$ is obtained by detection of the edges of the blood vessels conveying the object.

5. Method according to claim 3, **characterized in that**, in order to obtain the angle θ, said object is assimilated to a vertical rod with a finished height D and the angle θ is calculated from the following equation:

$$L = \frac{D}{\left| V_y - V_g \sin\theta \right|} \sqrt{V_y^2 + V_g^2 \cos^2\theta}$$

where L is the length of the ridge.

6. Method according to claim 5, **characterized in that** in the case where $V_g$*sin (θ) < $V_y$:

$$\tan\theta = \tan\alpha - \frac{D}{L\cos\alpha}$$

$$V_g = \left| V_y \right| \sqrt{\left(1 - \frac{D}{L\sin\alpha}\right)^2 + \frac{1}{\tan^2\alpha}}$$

7. Method according to claim 5, **characterized in that** in the case where $V_g$*sin(θ) > $V_y$:

$$\tan\theta = \tan\alpha + \frac{D}{L\cos\alpha}$$

$$V_g = \left| V_y \right| \sqrt{\left(1 + \frac{D}{L\sin\alpha}\right)^2 + \frac{1}{\tan^2\alpha}}$$

8. Method according to claim 3, **characterized in that** a second image of the same plane is acquired but in a reversed scanning, and the following equation is used:

$$L' = \frac{D}{\left| -V_y - V_g \sin\theta \right|} \sqrt{V_y^2 + V_g^2 \cos^2\theta}$$

where L' is the length of the ridge during the reversed scanning.

9. Method according to claim 8 and 5, **characterized in that** when $V_g$*sin(θ) $| < |V_y|$ :

$$\tan\theta = \tan\alpha \frac{L - L'}{L + L'}$$

$$V_g = |V_y| \sqrt{\left(\frac{L-L'}{L+L'}\right)^2 + \frac{1}{\tan^2 \alpha}}$$

where L is the length of the ridge in a first direction of scanning, and L' the length of the ridge during the reversed scanning.

**10.** Method according to claims 5 and 8, **characterized in that** when $|V_g*\sin(\theta)| > |V_y|$:

$$\tan\theta = \tan\alpha \frac{L+L'}{L-L'}$$

$$V_g = |V_y| \sqrt{\left(\frac{L+L'}{L-L'}\right)^2 + \frac{1}{\tan^2 \alpha}}$$

**11.** Method according to claim 3, **characterized in that** when $V_g*\sin(\theta) = V_y$, in order to determine $V_g$ and $\theta$ the following equation is also used:

$$V_g = \left|\frac{V_y}{\sin \alpha}\right|$$

**12.** Method according to claim 3, **characterized in that** as said object is a red blood cell, its form on the acquired image is assimilated to an ellipse with a radius R the angle $\alpha$ of which between the ridge and the "x" axis is given by:

$$\tan(2\alpha) = \frac{2\cos(\theta)}{\dfrac{V_g}{V_y} - 2\sin(\theta)}$$

and the length of the principal axis is given by:

$$L = \frac{2R\sqrt{2}}{\sqrt{V_r^2 - 2V_r \sin(\theta) + 2 - |V_r|\sqrt{V_r^2 - 4V_r \sin(\theta) + 4}}}$$

with $V_r = \dfrac{V_g}{V_y}$

**13.** Method according to any one of the preceding claims, **characterized in that** a confocal microscope is used.

**14.** Method according to any one of the preceding claims, **characterized in that** a light-scanning microscope in fibre mode is used.

**15.** Method according to any one of claims 1 to 13, **characterized in that** a non-fibre light-scanning microscope is used.

16. Light-scanning microscopy system, used to measure the velocity of a microscopic object moving inside a flow, such as a blood flow, this system implementing a method according to any one of the preceding claims; this system comprising:

- means for acquiring an image by x and y light scanning of a plane containing said object;
- means for detecting in the plane (x, y) a ridge produced by the movement of said object during the acquisition of said image;
- means for determining in the plane (x, y) the slope of said ridge; and
- means for estimating the velocity $V_g$ of said object from the thus-determined slope.

17. System according to claim 16, **characterized in that** during detection of the ridge, the system comprises:

- means for enhancing a group of ridges of the image by application of a filter;
- means for applying a threshold so as to retain the most significant ridges;
- means for fitting an ellipse on each of these ridges; and
- means for identifying said ridge.

18. System according to claim 16 or 17, **characterized in that** a confocal microscope is used.

19. System according to any one of claims 16 to 18, **characterized in that** a light-scanning microscope in fibre mode is used.

20. System according to any one of claims 16 to 18, **characterized in that** a non-fibre light-scanning microscope is used.

**Patentansprüche**

1. Verfahren zum Messen der Geschwindigkeit eines sich im Inneren einer Strömung, wie eines Blutstroms, bewegenden mikroskopischen Objekts, von einem Lichtabtastungsmikroskop aus, wobei dieses Verfahren die folgenden Schritte umfasst:

- Erfassung eines Bildes durch Lichtabtastung einer das Objekt enthaltenden Ebene in x und y;
- Erfassung eines durch die Bewegung des Objekts bei der Erfassung des Bildes erzeugten Streifens in der Ebene (x, y);
wobei das Verfahren **dadurch gekennzeichnet ist, dass** es die Schritte umfasst, die bestehen in:
- der Bestimmung der Neigung des Streifens in der Ebene (x, y); und
- der Schätzung der Geschwindigkeit $V_g$ des Objekts ausgehend von der auf diese Weise bestimmten Neigung.

2. Verfahren nach Anspruch 1, **dadurch gekennzeichnet, dass** der Schritt der Erfassung des Streifens die folgenden Schritte umfasst:

- Akzentuierung einer Einheit von Streifen des Bildes durch Verwendung eines Filters;
- Anwendung einer Schwelle, so dass die wichtigsten Streifen beibehalten werden;
- Anpassung einer Geraden oder einer Ellipse an jeden dieser Streifen; und
- Identifizierung des Streifens.

3. Verfahren nach Anspruch 1 oder 2, **dadurch gekennzeichnet, dass** die Geschwindigkeit $V_g$ des Objekts durch die folgende Gleichung gegeben ist:

$V_g * \cos(\theta) = V_y/\tan(\alpha)$, worin $V_y$ die vertikale Geschwindigkeit des für die Abtastung verwendeten Lichtflecks und "$\alpha$" der Winkel zwischen der horizontalen Achse "x" und dem Streifen ist.

4. Verfahren nach Anspruch 3, **dadurch gekennzeichnet, dass** der Winkel $\theta$ durch Erfassung der Ränder von das Objekt befördernden Blutgefäßen ist.

5. Verfahren nach Anspruch 3, **dadurch gekennzeichnet, dass** man, um den Winkel $\theta$ zu erhalten, das Objekt einem vertikalen Stab mit endlicher Höhe D gleichsetzt und den Winkel $\theta$ aus der folgenden Gleichung berechnet:

$$L = \frac{D}{\left|V_y - V_g \sin\theta\right|} \sqrt{V_y^2 + V_g^2 \cos^2\theta}$$

worin L die Menge des Streifens ist.

6.  Verfahren nach Anspruch 5, **dadurch gekennzeichnet, dass** in dem Fall, in dem $V_g \ast \sin(\theta) < V_y$:

$$\tan\theta = \tan\alpha - \frac{D}{L\cos\alpha}$$

$$V_g = \left|V_y\right| \sqrt{\left(1 - \frac{D}{L\sin\alpha}\right)^2 + \frac{1}{\tan^2\alpha}}$$

7.  Verfahren nach Anspruch 5, **dadurch gekennzeichnet, dass** in dem Fall, in dem $V_g \ast \sin(\theta) > V_y$:

$$\tan\theta = \tan\alpha + \frac{D}{L\cos\alpha}$$

$$V_g = \left|V_y\right| \sqrt{\left(1 + \frac{D}{L\sin\alpha}\right)^2 + \frac{1}{\tan^2\alpha}}$$

8.  Verfahren nach Anspruch 3, **dadurch gekennzeichnet, dass** man ein zweites Bild von derselben Ebene, jedoch in einer umgekehrten Abtastung, erfasst und man die folgende Gleichung verwendet:

$$L' = \frac{D}{\left|-V_y - V_g \sin\theta\right|} \sqrt{V_y^2 + V_g^2 \cos^2\theta} \quad .$$

worin L' die Länge des Streifens bei der umgekehrten Abtastung ist.

9.  Verfahren nach Anspruch 8 und 5, **dadurch gekennzeichnet, dass**, wenn $\left|V_g \ast \sin(\theta)\right| < \left|V_y\right|$:

$$\tan\theta = \tan\alpha \frac{L - L'}{L + L'}$$

$$V_g = \left|V_y\right| \sqrt{\left(\frac{L - L'}{L + L'}\right)^2 + \frac{1}{\tan^2\alpha}}$$

worin L die Länge des Streifens in einem ersten Abtastungssinn und L' die Länge des Streifens bei der umgekehrten Abtastung ist.

10. Verfahren nach Anspruch 5 und 8, **dadurch gekennzeichnet, dass**, wenn $|V_g * \sin(\theta)| > |V_y|$:

$$\tan\theta = \tan\alpha \, \frac{L + L'}{L - L'}$$

$$V_g = |V_y| \sqrt{\left(\frac{L + L'}{L - L'}\right)^2 + \frac{1}{\tan^2\alpha}}$$

11. Verfahren nach Anspruch 3, **dadurch gekennzeichnet, dass**, wenn $V_g * \sin(\theta) = V_y$, man für die Bestimmung von $V_g$ und $\theta$ außerdem die folgende Gleichung verwendet:

$$V_g = \left|\frac{V_y}{\sin\alpha}\right|$$

12. Verfahren nach Anspruch 3, **dadurch gekennzeichnet, dass**, wenn das Objekt ein rotes Blutkörperchen ist, man seine Form auf dem erfassten Bild einer Ellipse mit dem Radius R gleichsetzt, deren Winkel $\alpha$ zwischen dem Streifen und der Achse "x" gegeben ist durch:

$$\tan(2\alpha) = \frac{2\cos(\theta)}{\dfrac{V_g}{V_y} - 2\sin(\theta)}$$

und die Länge der Hauptachse gegeben ist durch:

$$L = \frac{2R\sqrt{2}}{\sqrt{V_r^2 - 2V_r\sin(\theta) + 2} - |V_r|\sqrt{V_r^2 - 4V_r\sin(\theta) + 4}}$$

$$\text{mit } V_r = \frac{V_g}{V_y}$$

13. Verfahren nach einem der vorhergehenden Ansprüche, **dadurch gekennzeichnet, dass** man ein konfokales Mikroskop verwendet.

14. Verfahren nach einem der vorhergehenden Ansprüche, **dadurch gekennzeichnet, dass** man ein Lichtabtastungsmikroskop im Fasermodus verwendet.

15. Verfahren nach einem der Ansprüche 1 bis 13, **dadurch gekennzeichnet, dass** man ein Nichtfaser-Lichtabtastungsmikroskop verwendet.

**16.** Lichtabtastungs-Mikroskopiesystem, das zum Messen der Geschwindigkeit eines sich im Inneren einer Strömung, wie eines Blutstroms, bewegenden mikroskopischen Objekts verwendet wird, wobei dieses System ein Verfahren nach einem der vorhergehenden Ansprüche einsetzt; wobei dieses System umfasst:

- Mittel zum Erfassen eines Bildes durch Lichtabtastung einer das Objekt enthaltenden Ebene in x und y;
- Mittel zum Erfassen eines durch die Bewegung des Objekts bei der Erfassung des Bildes erzeugten Streifens in der Ebene (x, y);
- Mittel zum Bestimmen der Neigung des Streifens in der Ebene (x, y); und
- Mittel zum Schätzen der Geschwindigkeit $V_g$ des Objekts ausgehend von der auf diese Weise bestimmten Neigung.

**17.** System nach Anspruch 16, **dadurch gekennzeichnet, dass** das System während der Erfassung des Streifens umfasst:

- Mittel zum Akzentuieren einer Einheit von Streifen des Bildes durch Anlegen eines Filters;
- Mittel zum Anlegen einer Schwelle, so dass die wichtigsten Streifen beibehalten werden;
- Mittel zum Anpassen einer Ellipse an jeden dieser Streifen; und
- Mittel zur Identifizierung des Streifens.

**18.** System nach Anspruch 16 oder 17, **dadurch gekennzeichnet, dass** man ein konfokales Mikroskop verwendet.

**19.** System nach einem der Ansprüche 16 bis 18, **dadurch gekennzeichnet, dass** man ein Lichtabtastungsmikroskop im Fasermodus verwendet.

**20.** System nach einem der Ansprüche 16 bis 18, **dadurch gekennzeichnet, dass** man Nichtfaser-Lichtabtastungs-mikroskop verwendet.

FIG.1

FIG.2

FIG.3

FIG.4

EP 1 740 974 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6473698 B

- WO 2004008952 A1

**Littérature non-brevet citée dans la description**

- **H. Wayland ; RC. Johnson.** Erythrocyte velocity measurement in microvessels by a two-slit photometric method. *J. Appl. Physiol.,* 1967, vol. 22 (2), 333-337
- **W. Groner ; I.W. Winkelman ; A.G. Harris ; G. Inde ; G.I. Bouma ; K. Messmer ; R.G. Nadeau.** Orthogonal Polarisation Spectral Imagina : A new method for study of microcirculation. *Nature Medecine,* 1999, vol. 5, 1209-1213
- **Yoshinobu Sato et al.** Measuring microcirculation using spatiotemporal image analysis. *CVRMed,* 1995, 302-308

- Two-photon imaging of neocortical microcirculation. **D. Kleinfeld ; W. Denk.** Imaging Neurons: A Laboratory Manual. Cold Spring Harbor Laboratory Press, 1999, 23.1-23.15
- **E. Chaigneau et al.** Two-photon imaging of capillary blood flow in olfactory bulb glomeruli. *PNAS,* 28 Octobre 2003, vol. 100 (22), 13081-13086, http://www.pnas.org/cgi/content/full/100/22/13081